# FASCICULE DE BREVET EUROPEEN

(11) **EP 3 723 717 B1**
(45) Date de publication et mention de la délivrance du brevet: **01.11.2023**
(21) Numéro de dépôt: 18833282.9
(22) Date de dépôt: 13.12.2018
(51) Int. Cl.: A61K 8/44, A61Q 1/02, A61Q 1/06, A61Q 1/10, A61K 8/81, A61K 8/06, A61K 8/42

(54) **ÉMULSION EAU-DANS-HUILE POUR LE SOIN OU LE MAQUILLAGE DE LA PEAU OU DES LÈVRES**
WASSER-IN-ÖL-EMULSION FÜR HAUT- ODER LIPPENPFLEGE ODER MAKEUP
WATER-IN-OIL EMULSION FOR SKIN OR LIP CARE OR MAKEUP

(30) Priorité: 14.12.2017 FR 1762186
(43) Date de publication de la demande: 21.10.2020
(73) Titulaire: LVMH Recherche, 45800 Saint Jean de Braye (FR)
(72) Inventeur: BROSSARD, Fabienne, 45000 ORLEANS (FR); DE LA POTERIE, Valérie, 45740 LAILLY EN VAL (FR)
(74) Mandataire: Cabinet Beau de Loménie
(86) Numéro de dépôt international: PCT/FR2018/053267
(87) Numéro de publication internationale: WO 2019/115958

(56) Documents cités:
- WO-A1-2016/039771
- US-A1- 2012 164 093

## Description

La présente invention concerne une émulsion eau-dans-huile comprenant un amide d'acide aminé et d'acide gras à longue chaîne, et un polymère portant des chaînes alkyles comprenant de 10 à 30 atomes de carbone. Cette émulsion est destinée au soin ou au maquillage de la peau ou des lèvres.

### ART ANTERIEUR

L'invention porte sur de nouvelles émulsions eau-dans-huile dotées de teintes, de qualités sensorielles et d'une tenue dans le temps qui n'ont jamais été obtenues simultanément car elles étaient jusqu'à présent incompatibles.

Par exemple, pour obtenir un rendu naturel dit également effet « nude », on formule les émulsions de telle sorte que peu de matière soit déposée sur la partie du corps voulue (peau ou lèvres par exemple). Or généralement, moins on applique de matière, moins le film formé est résistant aux frottements, à la transpiration ou aux mouvements de la peau, et moins les couleurs sont intenses. A l'opposé, si l'on cherche à améliorer la tenue ou l'adhésion du film, on risque d'en diminuer la souplesse et la douceur, car généralement on dépose plus de matière.

Il existe de nombreux produits pour le maquillage ou le soin des lèvres ou de la peau à base d'huiles. Ces produits peuvent être anhydres ou en émulsion.

Certains de ces produits, lorsqu'ils ne contiennent ni cire ni eau, ont un visuel attractif tel que la transparence ou la beauté des teintes. Mais ils n'apportent aucune sensation de fraîcheur à l'application, et souffrent d'une faible couvrance une fois appliqués.

Par ailleurs, les produits qui contiennent des cires pour apporter de la couvrance, présentent les inconvénients liés à la cristallinité des cires. Les teintes sont ternes et moins attractives ; le rendu maquillage est mat ; le dépôt laissé sur la peau ou les lèvres laisse la sensation d'un film cireux, inconfortable, lourd et présent ; les produits accrochent au moment de leur application qui manque ainsi de glissant.

Le besoin subsiste donc de combiner les avantages d'un produit anhydre avec la fraîcheur et la sensorialité d'une émulsion sans les inconvénients causés par les cires, nécessaires dans l'art antérieur pour obtenir un produit, sous la forme d'une pâte ou d'un stick, qui couvre suffisamment la peau et les lèvres.

Le besoin subsiste également de disposer d'une composition qui donnent des dépôts sur la peau ou les lèvres de faible épaisseur et de bonne tenue dans le temps. Lorsque la composition contient des pigments, le rendu de la couleur peut être intense malgré la faible quantité de matière déposée sur la peau ou les lèvres.

On cherche plus précisément à donner plus de consistance à un produit contenant des huiles, mais sans utiliser de cires comme moyen de structuration, le produit contenant par ailleurs de l'eau pour apporter de la fraîcheur. On cherche à substituer les cires qui réduisent la glisse du produit à l'application, qui forment un film inconfortable sur la peau ou les lèvres, et qui ternissent les teintes.

### DESCRIPTION GENERALE DE L'INVENTION

L'invention concerne donc une composition en émulsion rendue solide ou pâteuse, non pas par un réseau de cires cristallin, mais par le mélange d'un amide de l'acide glutamique et d'un polymère hydrocarboné, lequel mélange peut être solubilisé par au moins une huile non volatile. Cette composition allie de façon très surprenante sensorialité et performance.

### DESCRIPTION DETAILLEE

Selon un mode de réalisation, l'invention porte sur une composition cosmétique de soin ou de maquillage de la peau ou des lèvres sous la forme d'une émulsion eau-dans-huile contenant une phase grasse, un tensio-actif et de l'eau, caractérisée en ce que ladite phase grasse comprend au moins un amide de l'acide glutamique, ledit amide comprenant au moins un groupement alkoyle ayant de 6 à 14 atomes de carbone, et comprend au moins un (co)polymère vinylique alkylé comprenant au moins un substituant alkyle comprenant de 10 à 30 atomes de carbone, ladite phase grasse comprenant également au moins une première huile non volatile hydrocarbonée et au moins une deuxième huile non volatile comprenant au moins une fonction ester et/ou au moins une fonction alcool, et au moins une chaîne hydrocarbonée comprenant de 10 à 22 atomes de carbone.

La composition comprend de préférence une quantité de cire(s) inférieure à 5% en masse, et de préférence encore inférieure à 3% en masse, voire même inférieure à 2% en masse. La composition contient de préférence moins de 1% en masse de cire(s), et elle en est encore préférentiellement dépourvue.

La composition de l'invention ne présente pas les inconvénients mentionnés précédemment au sujet des produits anhydres et/ou comprenant des cires, notamment ceux qui se trouvent sous forme de pâtes, de sticks ou ceux qui sont coulés dans des godets.

Cette composition peut avantageusement former, après application sur la peau ou les lèvres, un dépôt de faible épaisseur dont l'effet cosmétique persiste dans le temps. Lorsque la composition contient des pigments, le rendu de la couleur peut être intense malgré la faible quantité de matière déposée sur la peau ou les lèvres.

Dans un mode de réalisation plus particulier, l'invention porte sur une composition de soin ou de maquillage dont la consistance peut être solide ou crémeuse, sous la forme d'une émulsion eau-dans-huile contenant une phase grasse, un tensio-actif et de l'eau, ladite phase grasse comprenant elle-même au moins une première huile non volatile hydrocarbonée et au moins une deuxième huile non volatile comprenant au moins une fonction ester ou alcool, lesquelles huiles sont miscibles, ladite composition comprenant en outre un polymère alkylé comprenant au moins un substituant alkyle comprenant de 10 à 30 atomes de carbone, et au moins un alkylamide de l'acide glutamique en une quantité suffisante pour obtenir le caractère solide, structuré ou crémeux de la composition.

Cette association d'ingrédients permet d'obtenir un produit dont l'application sur la peau ou les lèvres est très facile, rapide et surprenante par sa glisse extrême, et qui forme un film léger, confortable, intense en couleur et de longue tenue sur la peau ou les lèvres. L'invention permet d'obtenir à la fois une très bonne tenue avec un film léger, souple et donc très confortable. Toutes ces propriétés étaient jusqu'à présent inconciliables dans la formulation de produits structurés avec des cires, et n'avaient jamais été obtenues simultanément dans l'art antérieur. Notamment, les performances de longue tenue dans le temps d'un film déposé sur la peau ou les lèvres sont obtenues dans l'art antérieur mais avec des films présents et non confortables car ils contiennent des cires.

La sensorialité de la composition selon l'invention est très surprenante pour le consommateur car elle est à la fois très glissante, d'application facile en un seul passage, en apportant de la fraîcheur, de la tenue, du confort grâce à la finesse du film déposé, et une couleur intense.

La phase continue de l'émulsion de l'invention est une phase grasse dans laquelle au moins une des huiles qu'elle contient est structurée par un amide de l'acide glutamique (glutamide). Ces amides sont par conséquent des agents structurants de ladite huile, laquelle peut être choisie parmi les huiles hydrocarbonées, les huiles esters et les huiles alcools.

L'amide comprend de préférence au moins un groupement alkoyle comprenant de 6 à 14 atomes de carbone, par exemple 8 ou 12 atomes de carbone. Un tel amide de l'acide glutamique est par exemple décrit dans le brevet FR 2 820 739.

Le glutamide est de préférence choisi parmi le dibutyl lauroyl glutamide, le dibutyl ethylhexanoyl glutamide et leurs mélanges et peut être par exemple un des produits de marques EB-21, GP-1, AJK-OD2046, AJK-BG2055 et AJK-CE2046 fabriqués par la société Ajinomoto.

L'amide de l'acide glutamique est par exemple choisi parmi le dibutyl lauroyl glutamide et le dibutyl ethylhexanoyl glutamide. Selon un mode de réalisation, la composition contient le dibutyl lauroyl glutamide et le dibutyl ethylhexanoyl glutamide.

L'amide ou le mélange d'amides représente par exemple entre 0,1% et 15,0% en masse, entre 1,0% et 15,0 % en masse, entre 0,5% et 8,0% en masse, entre 1,0% et 5,0% en masse, entre 0,8% et 5% en masse, voire entre 2,0 et 3,0% en masse par rapport à la masse de la composition.

La composition de l'invention comprend au moins un (co)polymère vinylique alkylé. Celui-ci est de préférence non réticulé et solubilisé dans une des huiles de la phase grasse.

Il peut être obtenu par polymérisation d'un monomère comprenant au moins un groupement alkyle ayant de 10 à 30 atomes de carbone, ledit monomère étant choisi parmi un acide alkyl(méth)acrylique ou un de ses esters ; un (méth)acrylate d'alkyle ; et une alkyl-pyrrolidone.

Il peut être également obtenu par polymérisation d'un mélange de la vinylpyrrolidone et d'une alpha-oléfine comprenant de 14 à 22 atomes de carbone, de préférence de 16 à 20 atomes de carbone.

Selon un mode de réalisation, le (co)polymère vinylique alkylé est obtenu à partir d'un monomère (méth)acrylate d'alkyle dont le groupement alkyle comprend de 10 à 30 atomes de carbone, par exemple 14, 16, 18, 20 ou 22 atomes de carbone.

Dans un autre mode de réalisation le (co)polymère vinylique alkylé est issu de la polymérisation d'un premier monomère (méth)acrylate de stéaryle ou (méth)acrylate de béhényle, lequel monomère est greffé avec un groupement siliconé tel que par exemple le polydiméthylsiloxane, et éventuellement d'au moins un deuxième monomère choisi parmi l'acide (méth)acrylique, le méthacrylate de méthyle, le méthacrylate de butyle et l'acrylate de 2-éthylhexyle. Il peut s'agir par exemple de composés de noms INCI ACRYLATES/BEHENYL ACRYLATE/DIMETHICONE METHACRYLATE COPOLYMER ou nom INCI ACRYLATES/STEARYL ACRYLATE/DIMETHICONE METHACRYLATE COPOLYMER, et de produits commerciaux de références KP-561P ou KP-562P vendus par la société Shin Etsu.

L'alkyl-pyrrolidone dont le groupement alkyle comprend de 10 à 30 atomes de carbone a de préférence un groupement alkyle comprenant 30 atomes de carbone (nom INCI TRIACONTANYL PVP).

Le (co)polymère vinylique alkylé peut être également issu de la polymérisation du mélange de la vinylpyrrolidone et d'une alpha-oléfine comprenant de 14 à 22 atomes de carbone, de préférence comprenant de 16 à 20 atomes de carbone, et par exemple le 1-éicosène (nom INCI VP/EICOSENE COPOLYMER) ou le 1-hexadécène (nom INCI VP/HEXADECENE COPOLYMER). De tels produits sont commercialisés sous les marques Antaron^{®} ou Unimer^{®}.

Le (co)polymère vinylique alkylé peut enfin être un homopolymère d'un (méth)acrylate d'alkyle dont le groupement alkyle comprend de 10 à 30 atomes de carbone tels que les produits de marque Intelimer^{®}, ou de nom INCI C10-C30 ALKYL ACRYLATE.

Le (co)polymère vinylique alkylé peut représenter entre 1% et 30% en masse de la masse de la composition, selon l'usage auquel on la destine. Le (co)polymère représente ainsi par exemple entre 15% et 25% en masse pour un rouge à lèvres, entre 5 et 10% en masse pour un eye liner et entre 1 et 5% en masse pour un fond de teint.

Le (co)polymère vinylique peut être choisi parmi les composés portant la dénomination INCI ACRYLATES/DIMETHICONE METHACRYLATE COPOLYMER, ACRYLATES/BEHENYL ACRYLATE/DIMETHICONE METHACRYLATE COPOLYMER, ACRYLATES/STEARYL ACRYLATE/DIMETHICONE METHACRYLATE COPOLYMER, C10-C30 ALKYL ACRYLATE, VP/EICOSENE COPOLYMER, VP/HEXADECENE COPOLYMER OU TRIACONTANYL PVP.

Dans plusieurs modes de réalisation, la composition contient de 1 à 15% en masse de TRIACONTANYL PVP, de 15 à 25% en masse d'un ACRYLATES/STEARYL ACRYLATE/METHACRYLATE DIMETHICONE, ou de 15 à 25% en masse d'un mélange de VP/EICOSENE et d'un ACRYLATES/STEARYL ACRYLATE/METHACRYLATE DIMETHICONE.

La composition peut contenir tout type d'huile volatile ou non volatile connu de l'homme du métier, comme une huile siliconée, une huile hydrocarbonée ou une huile comprenant carbone, hydrogène et oxygène.

La phase grasse comprend au moins une première huile non volatile hydrocarbonée et au moins une deuxième huile non volatile comprenant au moins une fonction ester et/ou au moins une fonction alcool, et au moins une chaîne hydrocarbonée comprenant de 10 à 22 atomes de carbone, dite ci-après « huile ester ou alcool ».

Selon un mode de réalisation, on préfère utiliser une faible quantité d'huiles siliconées (huile comprenant au moins un atome de silicium), qui nuisent parfois à la stabilité de la composition et à sa bonne conservation. Dans un mode de réalisation préféré, la composition contient moins de 3% en masse, de préférence moins de 1% en masse d'une huile siliconée. Elle en est préférentiellement dépourvue.

Parmi les huiles esters ou alcools, on préfère les huiles constituées d'atomes de carbone, oxygène et hydrogène comprenant au moins une chaîne hydrocarbonée comprenant de 10 à 22 atomes de carbone linéaire ou ramifiée, saturée ou insaturée, par exemple les huiles choisies parmi l'isostéaryl isotéarate, les alcools linéaires ou ramifiés saturés liquides tels que l'octyldodécanol, les esters de dimères de l'acide dilinoléique (tel que le produit de dénomination INCI DIMER DILINOLEYL DIMER DILINOLEATE dont une référence commerciale est le Lusplan^{®} DD DA7), le pentaerythrityl triisostéarate, le triméthylolpropane triisostéarate, le 2-octyldodécyl-12 stéaroyle stéarate (par exemple l'huile de référence commerciale Ceraphyl^{®} 847).

Parmi les huiles hydrocarbonées, on préfèrera les huiles choisies parmi les polybutènes, les polyisobutènes hydrogénés, les polydécènes hydrogénés, l'huile minérale encore appelée paraffine liquide (de noms INCI MINERAL OIL ou PARAFFINUM LIQUIDUM, par exemple l'huile minérale de marque Primol^{®} 352). Une huile hydrocarbonée est définie conformément aux connaissances générales de l'homme du métier et peut être une huile constituée de carbone et d'hydrogène de type alcane ou alcène.

L'huile ester ou alcool, lorsqu'elle est présente dans la composition, est de préférence miscible avec l'huile hydrocarbonée de manière à ce que la phase grasse soit structurée de façon optimale par l'amide, de préférence le glutamide.

Le rapport massique entre la première huile non volatile et la deuxième huile non volatile est de préférence compris entre 1/3 et 3/1, de préférence entre 1/1 et 3/1.

La composition peut contenir, outre les huiles mentionnées précédemment au moins une huile volatile, qui peut être choisie parmi l'isododécane, l'isohexadecane, les cocoate alkanes (par exemple les produits de référence commerciale Vegelights^{®}) et la cyclopentasiloxane. La quantité d'huile(s) volatile(s) peut être comprise entre 5 et 15% en masse.

La composition peut comprendre de 8 à 40% en masse d'une première huile non volatile hydrocarbonée, de 8 à 12% en masse d'une huile volatile et de 8 à 40% en masse d'une huile deuxième huile ester ou alcool.

Selon un mode de réalisation, la composition comprend de 15 à 40% en masse d'une première huile non volatile hydrocarbonée, de 8 à 12% en masse d'une huile volatile et de 8 à 15% en masse d'une deuxième huile non volatile ester ou alcool.

Selon un autre mode de réalisation, la composition comprend de 8 à 15% en masse d'une première huile non volatile hydrocarbonée, de 8 à 12% en masse d'une huile volatile et de 15 à 40% en masse d'une deuxième huile non volatile ester ou alcool.

La composition de l'invention peut être utilisée pour le de soin ou de maquillage de la peau ou des lèvres.

La composition de l'invention peut être utilisée pour fabriquer des produits de maquillage pour les lèvres, pour la peau (fond de teint, fard à paupière ou fard à joues, produits de soin protecteurs des UV et des produits de soin perfecteurs).

Elle peut prendre diverses formes telles qu'un sérum, une lotion, une crème ou un hydrogel, un masque, un stick ou un patch. Selon un mode de mise en oeuvre, la composition est solide.

Selon un mode de réalisation, la composition est solide au sens où l'entend un homme du métier. Dans certains modes de réalisation, il n'est pas nécessaire de la mettre dans un contenant pour être conservée. Elle peut néanmoins nécessiter un applicateur.

Une composition qui n'est pas solide est par exemple un produit en flacon, en bouillote ou en bouteille tel qu'un mascara appliqué avec une brosse, un gloss appliqué avec un embout mousse, un fond de teint en flacon, une lotion de soin ou une crème de soin, ces exemples n'étant pas limitatifs.

La composition de l'invention peut être obtenue par mélange des ingrédients non volatiles de la phase grasse à une température suffisante pour la faire fondre, par introduction de la phase aqueuse préalablement chauffée dans la phase grasse fondue, par ajout de la phase pigmentaire contenant des pigments mais aussi des charges puis par ajout éventuel d'une huile volatile, pour obtenir un mélange fluide.

Selon un mode de réalisation, la composition est sous forme solide. Dans ce cas, elle peut être coulée et/ou moulée, i.e. obtenue par un procédé comprenant une étape de préparation du mélange fluide décrite précédemment, une étape de coulage du mélange fluide dans un contenant, une étape de refroidissement (composition coulée), et éventuellement une étape de démoulage (composition coulée et moulée). Le contenant peut être un godet de faible épaisseur ou un moule cylindrique.

Selon un autre mode de réalisation, la composition est sous la forme d'une pâte dite également sous forme déstructurée. Dans ce cas, le mélange fluide est refroidit sous agitation jusqu'à température ambiante.

Ainsi, la présente invention a pour objet un procédé de fabrication d'une composition telle que décrite précédemment qui comprend au moins les étapes suivantes :
- l'eau et le tensio-actif sont mélangés et chauffés pour obtenir une phase aqueuse,
- la phase grasse contenant l'amide et le (co)polymère vinylique alkylé est fondue,
- la phase aqueuse est introduite dans la phase grasse fondue afin d'obtenir un mélange fluide, et
- le mélange fluide est laissé à refroidir jusqu'à température ambiante pour obtenir une composition sous forme solide ou crémeuse.

Selon une première variante, le mélange fluide est versé dans un moule avant d'être laissé à refroidir, puis démoulée pour obtenir la composition de l'invention sous forme solide.

Selon une deuxième variante, le mélange fluide est laissé à refroidir jusqu'à température ambiante sous agitation continue, pour obtenir la composition de l'invention sous forme d'une pâte, que l'on peut ensuite versée dans un conditionnement.

La composition peut être sous la forme d'un stick tel qu'un rouge à lèvres, un baume pour les lèvres, un stick correcteur, un fond de teint, un fard à joues ou un fard à paupières.

Le stick est de préférence sous la forme d'un cylindre pouvant avoir un diamètre standard de 11,8 mm, 12,1 mm, 12,7 mm, 9,5 mm ou 11,6 mm.

Dans l'émulsion eau-dans-huile de l'invention, l'eau représente de préférence entre 10% et 35% en masse de la masse de la composition.

La composition peut comprendre des polymères filmogènes qui sont dispersés dans l'eau ou solubilisés dans l'eau. Selon un mode de réalisation, elles en sont dépourvues.

La composition peut également contenir d'autres ingrédients qui participent à la stabilité et aussi à la tenue du produit, tels que des corps gras à consistance pâteuse à 25°C et des charges telles que la silice, le PMMA, les fluorophlogopites ou le nylon.

Elle peut comprendre en outre au moins un excipient cosmétiquement acceptable choisi parmi des pigments, des actifs, des colorants, des polymères additionnels, des agents de rhéologie additionnels, des parfums, des électrolytes, des ajusteurs de pH, des agents anti-oxydants, des conservateurs, des agents hydratants, des agents humectants, des filtres solaires organiques ou minéraux, ou des nacres.

Les composés sous forme particulaire (poudres) tels que les pigments et les charges représentent de préférence entre 10% et 30% en masse de la masse de la composition, selon la teinte que l'on souhaite obtenir.

Le tensio-actif est de préférence un tensio-actif susceptible de stabiliser des émulsions eau-dans-huile choisi parmi les composés ayant comme dénomination INCI le PEG-30 DIPOLYHYDROXYSTÉARATE (par exemple de marque Cithrol^{®} DPHS-SO), le DISTEARDIMONIUM HECTORITE AND POLYGLYCERYL-6 POLYRICINOLEATE AND POLYGLYCERYL-2 ISOSTÉARATE (par exemple de marque Nikkomulese^{®} WO-NS) et le SORBITAN SESQUIOLEATE (par exemple la marque Span^{®} 83). Il représente notamment entre 0,5% et 5% en masse de la masse de la composition.

L'invention décrit également un procédé cosmétique de soin ou de maquillage de la peau ou des lèvres comprenant l'application d'une composition telle que décrite précédemment.

### DESCRIPTION DE LA FIGURE

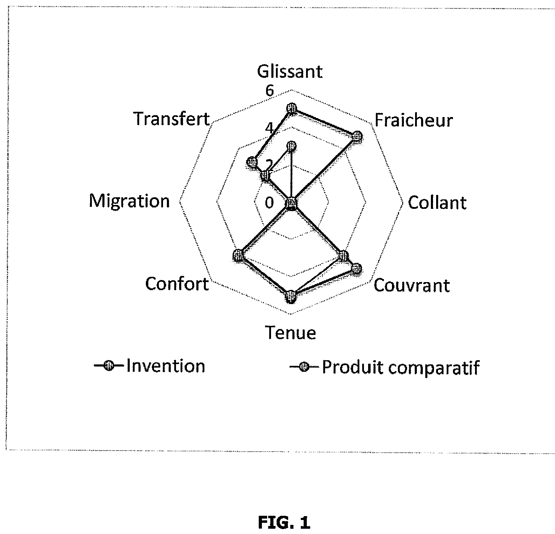

La Figure 1 présente le résultat d'une évaluation des propriétés sensorielles du rouge à lèvres selon l'invention de l'Exemple 2.

L'invention est illustrée par les exemples suivants.

### EXEMPLE 1 : COMPOSITIONS DE L'INVENTION

La composition de produits selon l'invention est décrite dans les tableaux suivants. Les pourcentages sont exprimés en masse.

### • Rouge à Lèvres

| Phase | Nom INCI | % |
|---|---|---|
| A | DIBUTYL LAUROYL GLUTAMIDE | 1.3 |
| A | DIBUTYL ETHYLHEXANOYL GLUTAMIDE | 0.9 |
| A | OCTYLDODECANOL | 8.8 |
| A | HYDROGENATED POLYISOBUTENE | 16.7 |
| A | VP/EICOSENE COPOLYMER | 10.0 |
| A | ACRYLATES/STEARYL ACRYLATE/DIMETHICONE MÉTHACRYLATE COPOLYMER | 10.0 |
| A | PEG-30 DIPOLYHYDROXYSTÉARATE | 3.5 |
| B | AQUA | Qsp 100 |
| B | Conservateurs | 2.9 |
| C | PIGMENTS | 10.0 |
| D | SILICA | 5.0 |
| D | SILICON DIOXIDE | 5.0 |
| E | ISODODECANE | 10.0 |

### Procédé de Préparation

- On chauffe au bain marie et sous agitation la phase A à 90-95°C et on homogénéise jusqu'à fonte complète.
- On chauffe au bain marie et sous agitation la phase B à 85°C.
- On verse la phase B dans la phase A, et on homogénéise quelques minutes sous agitation.
- La phase pigmentaire C puis les différentes charges D sont ajoutées successivement.
- On ajoute la phase E.
- La composition ainsi obtenue est coulée entre 85°C et 90°C directement dans un mécanisme de rouge à lèvres de diamètre intérieur compris entre 9 et 12 mm.
- On laisse la composition dans le mécanisme revenir à température ambiante.
- On démoule après environ 1h lorsque la composition est solide.

### • Fond de Teint

| Phase | Nom INCI | % |
|---|---|---|
| A | DIBUTYL LAUROYL GLUTAMIDE | 1.7 |
| A | DIBUTYL ETHYLHEXANOYL GLUTAMIDE | 1.1 |
| A | OCTYLDODECANOL | 11.0 |
| A | HYDROGENATED POLYDECENE | 19.0 |
| A | ETHYLHEXYL METHOXYCINNAMATE | 5.5 |
| A | DIETHYLAMINO HYDROXYBENZOYL HEXYL BENZOATE | 1.1 |
| A | BIS-ETHYLHEXYLOXYPHENOL METHOXYPHENYLTRIAZINE | 1.1 |
| A | TRIACONTANYL PVP | 3.2 |
| A | PEG-30 DIPOLYHYDROXYSTÉARATE | 3.7 |
| B | AQUA | Qsp 100 |
| B | PROPANEDIOL | 5.2 |
| B | Conservateurs | 3.0 |
| C | PIGMENTS | 11.0 |
| D | ISODODECANE | 10.5 |
| E | TITANIUM DIOXIDE | 4.0 |

### Procédé de préparation

Les phases A à C sont préparées et mélangées selon le même procédé que celui utilisé pour fabriquer le rouge à lèvres ci-dessus, les phases D et E sont ajoutées en final puis la composition obtenue est coulée à chaud en godet puis laissée à refroidir à température ambiante, ou laissée à refroidir sous agitation jusqu'à température ambiante pour obtenir une pâte.

### • Eve Liner

| Phase | Nom INCI | % massique |
|---|---|---|
| A | DIBUTYL LAUROYL GLUTAMIDE | 1.3 |
| A | DIBUTYL ETHYLHEXANOYL GLUTAMIDE | 0.8 |
| A | OCTYLDODECANOL | 8.8 |
| A | HYDROGENATED POLYISOBUTENE | 16.9 |
| A | ISOSTEARYL ISOSTÉARATE | 5.0 |
| A | TRIACONTANYL PVP | 10.0 |
| A | PEG-30 DIPOLYHYDROXYSTÉARATE | 3.5 |
| B | AQUA | Qsp 100 |
| B | PROPANEDIOL | 5.0 |
| B | Conservateurs | 2.9 |
| C | CI 77499 (IRON OXIDE) | 9.7 |
| D | SILICA | 3.0 |
| E | ISODODECANE | 10.0 |

### Procédé de préparation

Les phases A à E sont préparées et mélangées selon le même procédé que celui utilisé pour fabriquer le rouge à lèvres de l'exemple 1, à la différence près que le mélange final obtenu est coulé à chaud directement dans les mécanismes de stylo de diamètre compris entre 1 et 5 mm.

### EXEMPLE 2: Rouge à Lèvres de l'art antérieur contenant des cires et Rouge à Lèvres de l'invention

### Rouge à Lèvres selon l'invention

La composition est une émulsion eau-dans-huilede formule suivante (% exprimés en masse) :

| Phase | Nom INCI | % |
|---|---|---|
| A | DIBUTYL ETHYLHEXANOYL GLUTAMIDE | 0.8 |
| A | DIBUTYL LAUROYL GLUTAMIDE | 1.3 |
| A | OCTYLDODECANOL | 8.8 |
| A | HYDROGENATED POLYDECENE | 16.6 |
| A | PEG-30 DIPOLYHYDROXYSTÉARATE | 3.5 |
| A | ACRYLATES/STEARYL ACRYLATE/DIMETHICONE MÉTHACRYLATE COPOLYMER | 20.0 |
| B | AQUA | Qsp 100 |
| B | Conservateurs | 2.9 |
| C | PIGMENTS | 9.9 |
| D | SILICON DIOXIDE | 10.0 |
| E | ISODODECANE | 10.0 |

La composition est moulée en stick selon le procédé décrit à l'Exemple 1.

### Rouge à Lèvres comparatif

La composition comparative est de formule suivante (% exprimés en masse) :

| Phase | Nom INCI | % |
|---|---|---|
| A | POLYGLYCERYL-2 TRIISOSTEARATE | 23.3 |
| A | TRIMETHYLOLPROPANE TRIISOSTÉARATE | 11.0 |
| A | JOJOBA ESTERS | 10.1 |
| A | POLYETHYLENE | 9.5 |
| A | CERA MICROCRISTALLINA | 8.3 |
| A | ACRYLATES/STEARYL ACRYLATE/DIMETHICONE MÉTHACRYLATE COPOLYMER | 5.0 |
| A | HYDROGENATED POLYISOBUTENE | 2.5 |
| B | PIGMENTS | 11.3 |
| C | ISODODECANE | 19.0 |
| | TOTAL | 100.00 |

### Evaluation des propriétés de tenue

### - Protocole d'évaluation :

On a comparé la tenue du rouge à lèvres de l'invention et du rouge à lèvres comparatif. On a mesuré l'épaisseur du film *in vivo* sur une durée totale de 6 heures.

Chaque mesure correspond à la moyenne de trois mesures réalisées sur trois sticks de composition identique.
Panel de dix personnes (femmes de 20 à 50 ans)

Les mesures d'épaisseur du film sont réalisées à T0, T4H et T6H pour chacun des deux produits. On calcule ensuite le pourcentage (T0-T4H)/T0 et le pourcentage (T0-T6H)/T0.

### - Résultats :

Les résultats sont présentés dans le Tableau 1 ci-dessous.

**Tableau 1 - Mesure de la tenue des rouges à lèvres**

| | **Diminution de l'épaisseur du film** | |
|---|---|---|
| **Durée** | Invention | Comparatif |
| T+4h | -1% | 0% |
| T+6h après déjeuner | -6% | -11% |

L'épaisseur du film résiduel sur les lèvres de la composition selon l'invention est significativement plus importante que la composition comparative contenant des cires.

### Evaluation des propriétés sensorielles

### - Protocole d'évaluation :

Huit descripteurs sensoriels sont utilisés pour décrire le ressenti à l'application de la composition sur les lèvres
Panel de dix personnes (femmes de 20 à 50 ans)
Echelle : notes de 0 à 6 => 0 = Très médiocre / 6 = Excellent

### - Résultats :

Les résultats sont présentés dans le tableau 2 ci-dessous et sur la Figure 1.

**Tableau 2 - Propriétés sensorielles des rouges à lèvres**

| **Propriété** | **Invention** | **Comparatif** |
|---|---|---|
| Glissant | 5 | 3 |
| Fraîcheur | 5 | 0 |
| Collant | 0 | 0 |
| Couvrant | 5 | 4 |
| Tenue | 5 | 5 |
| Confort | 4 | 4 |
| Migration | 0 | 0 |
| Transfert | 3 | 2 |

La composition selon l'invention est significativement plus fraîche et plus glissante que la composition comparative contenant des cires.

### EXEMPLE 3 : Rouge à Lèvres selon l'invention

La composition selon l'invention est préparée avec les ingrédients suivants (% exprimés en masse).

| **Phase** | **Nom INCI** | **%** |
|---|---|---|
| A | DIBUTYL LAUROYL GLUTAMIDE | 1,56 |
| A | DIBUTYL ETHYLHEXANOYL GLUTAMIDE | 1,0 |
| A | OCTYLDODECANOL | 10,4 |
| A | HYDROGENATED POLYDECENE | 33,0 |
| A | TRICONTANYL PVP COPOLYMER | 5,0 |
| A | BIS-BEHENYL/ISOSTEARYL/PHYTOSTERYL DIMER DILINOLEYL DIMER DILINOLEATE | 1,0 |
| A | PEG-30 DIPOLYHYDROXYSTÉARATE | 3,5 |
| B | AQUA | Qsp 100 |
| B | Conservateurs | 2,9 |
| C | PIGMENTS | 10,0 |
| D | SILICA | 2,5 |
| D | METHYL METHACRYLATE CROSSPOLYMER | 2,5 |
| E | ISODODECANE | 10.0 |

### Procédé de préparation

Le procédé de préparation est identique à celui décrit dans l'Exemple 1.

### Evaluation des propriétés sensorielles

Les propriétés sensorielles du rouge à lèvres de l'invention et d'un rouge à lèvres sous forme d'un stick anhydre contenant des cires (Produit de la Fiche Mintel N°4601375) ont été évaluées par un panel d'experts entraînés à l'analyse sensorielle des rouges à lèvres.

La texture, l'efficacité immédiate et le résultat maquillage ont été plus particulièrement notés.

### Protocole d'évaluation :

L'étude a été réalisée sur un panel 17 femmes (âgées de 20 à 50 ans) qui se sont rendues de façon hebdomadaire dans une salle d'analyse sensorielle. Les conditions d'évaluation étaient standardisées par usage de descripteurs portant sur le ressenti à l'application et le résultat maquillage des deux produits à évaluer et à comparer. La gestuelle d'application était également précisée.

La salle d'analyse sensorielle était contrôlée en température et en hygrométrie, et équipée de cabines d'évaluations individuelles.

Chaque membre du panel a évalué le rouge à lèvres de l'invention et celui du marché par la notation de huit descripteurs sensoriels prédéfinis et communs à tout le panel qui étaient relatifs au glissant, à la couvrance, à la brillance et au confort. Ils ont attribué une note située sur une échelle allant de 0 (très médiocre) à 10 (excellent) pour chacun des descripteurs, et seules les moyennes significatives (alpha inférieur ou égal à 5%) ont été retenues pour effectuer l'analyse des résultats.

Les produits étaient codés par 3 chiffres pour pouvoir être testés en aveugle.

### Résultats :

Au moment de l'application du rouge à lèvres de l'invention sur les lèvres, la sensation est plus glissante que celle du rouge à lèvres de l'art antérieur dépourvu d'eau et contenant des cires. La sensation est plus glissante à l'entame (7,9/10 versus 2,8/10) et lors des différents passages (8,3/10 versus 3,7/10).

Le rouge à lèvres est également noté, de façon cohérente, moins adhérent (4,4/10 versus 8,2/10 pour le produit comparatif).

Le rouge à lèvres est donc plus glissant à l'utilisation, que ce soit au moment de l'entame ou au cours de l'application.

Le rouge à lèvres de l'invention laisse les lèvres plus souples immédiatement après application (7,2/10 contre 5,1/10), mais aussi au cours de la journée (7,6/10 contre 6,0/10). Au cours de la journée, le confort est perçu comme supérieur (7,8/10 contre 5,9/10). Le film est plus brillant (3/10 versus 0/10) et moins présent (4,5/10 contre 5,5/10).

Ces améliorations ne nuisent ni à la couvrance, ni à la tenue d'un film de produit de l'invention sur les lèvres. La couvrance du produit de l'invention est égale à celle du produit de l'art antérieur, alors qu'il ne contient pas de cire, ce qui est très surprenant (couvrance de 8,6/10 contre 8,9/10). La tenue, bien que très légèrement inférieure à celle du produit contenant des cires, reste très satisfaisante (7,5/10 pour l'invention et 8,5/10 pour le produit comparatif). Elle est bien supérieure à la tenue moyenne des produits de maquillages des lèvres sans cire.

## Revendications

1. Composition cosmétique de soin ou de maquillage de la peau ou des lèvres sous la forme d'une émulsion eau-dans-huile contenant une phase grasse, un tensio-actif et de l'eau, **caractérisée en ce que** ladite phase grasse comprend au moins un amide de l'acide glutamique, ledit amide comprenant au moins un groupement alkoyle ayant de 6 à 14 atomes de carbone, et comprend au moins un (co)polymère vinylique alkylé comprenant au moins un substituant alkyle comprenant de 10 à 30 atomes de carbone, ladite phase grasse comprenant également au moins une première huile non volatile hydrocarbonée et au moins une deuxième huile non volatile comprenant au moins une fonction ester et/ou au moins une fonction alcool, et au moins une chaîne hydrocarbonée comprenant de 10 à 22 atomes de carbone.

2. Composition selon la revendication 1, **caractérisée en ce que** l'amide de l'acide glutamique est choisi parmi le dibutyl lauroyl glutamide et le dibutyl ethylhexanoyl glutamide.

3. Composition selon la revendication 1 ou 2, **caractérisée en ce que** l'amide ou le mélange d'amides représente entre 0,1% et 15,0%, de préférence entre 0,5% et 8%, et de préférence encore entre 0,8% et 5% en masse de la masse de la composition.

4. Composition selon l'une des revendications précédentes, **caractérisée en ce que** le (co)polymère vinylique alkylé est obtenu par polymérisation d'un monomère comprenant au moins un groupement alkyle ayant de 10 à 30 atomes de carbone, ledit monomère étant choisi parmi un acide alkyl(méth)acrylique ou un de ses esters ; un (méth)acrylate d'alkyle ; et une alkyl-pyrrolidone.

5. Composition selon l'une des revendications précédentes, **caractérisée en ce que** le nom INCI du (co)polymère vinylique alkylé est choisi parmi ACRYLATES/DIMETHICONE METHACRYLATE COPOLYMER, ACRYLATES/BEHENYL ACRYLATE/DIMETHICONE METHACRYLATE COPOLYMER, ACRYLATES/STEARYL ACRYLATE/DIMETHICONE METHACRYLATE COPOLYMER, C10-C30 ALKYL ACRYLATE, VP/EICOSENE COPOLYMER, VP/HEXADECENE COPOLYMER ET TRIACONTANYL PVP.

6. Composition selon l'une des revendications précédentes, **caractérisée** en ce le (co)polymère vinylique alkylé représente entre 1% et 30% en masse de la masse de la composition.

7. Composition selon l'une des revendications 1 à 6, **caractérisée en ce que** le rapport massique entre la première huile non volatile et la deuxième huile non volatile est compris entre 1/3 et 3/1.

8. Composition selon l'une des revendications 1 à 7, **caractérisée en ce qu'**elle comprend de 15 à 20% en masse de la première huile non volatile, de 8 à 15% en masse de la deuxième huile non volatile, et de 8 à 12% en masse d'une huile volatile.

9. Composition selon l'une des revendications 1 à 8, **caractérisée en ce que** l'eau représente de 10% à 35% en masse de la masse de la composition.

10. Composition selon l'une des revendications 1 à 9, **caractérisée en ce qu'**elle comprend une quantité de cire(s) inférieure à 5% en masse.

11. Composition selon l'une des revendications 1 à 10, **caractérisée en ce qu'**elle est sous la forme d'un stick tel qu'un rouge à lèvres, un baume pour les lèvres, un stick correcteur, un fond de teint, un fard à joues ou un fard à paupières.

12. Procédé cosmétique de soin ou maquillage de la peau ou des lèvres comprenant l'application d'une composition selon l'une des revendications 1 à 11.

13. Procédé de fabrication d'une composition selon l'une des revendications 1 à 11, **caractérisé en ce qu'**il comprend au moins les étapes suivantes :
- l'eau et le tensio-actif sont mélangés et chauffés pour obtenir une phase aqueuse,
- la phase grasse contenant l'amide et le (co)polymère vinylique alkylé est fondue,
- la phase aqueuse est introduite dans la phase grasse fondue afin d'obtenir un mélange fluide, et
- le mélange fluide est laissé à refroidir jusqu'à température ambiante pour obtenir une composition sous forme solide ou crémeuse.

## Patentansprüche

1. Kosmetische Zusammensetzung für Haut- oder Lippenpflege oder -Makeup in der Form einer Öl-in-Wasser-Emulsion, die eine Fettphase, ein Tensid und Wasser enthält, **dadurch gekennzeichnet, dass** die Fettphase mindestens ein Amid von Glutaminsäure umfasst, das Amid mindestens eine Alkoylgruppe mit 6 bis 14 Kohlenstoffatomen umfasst und mindestens ein Alkylvinyl-(Co)-Polymer umfasst, das mindestens einen Alkylsubstituenten umfasst, der 10 bis 30 Kohlenstoffatome umfasst, wobei die Fettphase auch mindestens ein erstes nichtflüchtiges Kohlenwasserstofföl und mindestens ein zweites nichtflüchtiges Öl umfasst, das mindestens eine Esterfunktion und/oder mindestens eine Alkoholfunktion und mindestens eine Kohlenwasserstoffkette umfasst, die zwischen 10 und 22 Kohlenstoffatome umfasst.

2. Zusammensetzung nach Anspruch 1, **dadurch gekennzeichnet, dass** das Amid von Glutaminsäure aus Dibutyl-Lauroyl-Glutamid und Dibutyl-Ethylhexanoyl-Glutamid ausgewählt ist.

3. Zusammensetzung nach Anspruch 1 oder 2, **dadurch gekennzeichnet, dass** das Amid oder das Gemisch von Amiden zwischen 0,1 und 15,0, vorzugsweise zwischen 0,5 und 8 und vorzugsweise auch zwischen 0,8 und 5 Massenprozent der Masse der Zusammensetzung darstellt.

4. Zusammensetzung nach einem der vorhergehenden Ansprüche, **dadurch gekennzeichnet, dass** das Aklylvinyl-(Co)-Polymer durch Polymerisation eines Monomers erhalten wird, das mindestens eine Alkylgruppe mit 10 bis 30 Kohlenstoffatomen umfasst, wobei das Monomer aus einer Alkyl(meth)acrylsäure oder einem ihrer Ester, einem Alkyl(meth)acrylat und einem Alkylpyrrolidon ausgewählt ist.

5. Zusammensetzung nach einem der vorhergehenden Ansprüche, **dadurch gekennzeichnet, dass** die INCI-Bezeichnung des Alkylvinyl-(Co)-Polymers ausgewählt ist aus ACRYLATES/DIMETHICONE METHACRYLATE COPOLYMER, ACRYLATES/BEHENYL ACRYLATE/DIMETHICONE METHACRYLATE COPOLYMER, ACRYLATES/STEARYL ACRYLATE/DIMETHICONE METHACRYLATE COPOLYMER, C10-30 ALKYL ACRYLATE, VP/EICOSENE COPOLYMER, VP/HEXADECENE COPOLYMER und TRIACONTANYL PVP.

6. Zusammensetzung nach einem der vorhergehenden Ansprüche, **dadurch gekennzeichnet, dass** das Akylvinyl-(Co)-Polymer zwischen 1 und 30 Massenprozent der Masse der Zusammensetzung darstellt.

7. Zusammensetzung nach einem der Ansprüche 1 bis 6, **dadurch gekennzeichnet, dass** das Massenverhältnis zwischen dem ersten nichtflüchtigen Öl und dem zweiten nichtflüchtigen Öl zwischen 1:3 und 3:1 beträgt.

8. Zusammensetzung nach einem der Ansprüche 1 bis 7, **dadurch gekennzeichnet, dass** sie zwischen 15 und 20 Massenprozent von dem ersten nichtflüchtigen Öl, zwischen 8 und 15 Massenprozent von dem zweiten nichtflüchtigen Öl und zwischen 8 und 12 Massenprozent von einem flüchtigen Öl umfasst.

9. Zusammensetzung nach einem der Ansprüche 1 bis 8, **dadurch gekennzeichnet, dass** das Wasser zwischen 10 und 35 Massenprozent der Masse der Zusammensetzung darstellt.

10. Zusammensetzung nach einem der Ansprüche 1 bis 9, **dadurch gekennzeichnet, dass** sie eine Menge an Wachs(en) von unter 5 Massenprozent umfasst.

11. Zusammensetzung nach einem der Ansprüche 1 bis 10, **dadurch gekennzeichnet, dass** sie die Form eines Stifts, wie beispielsweise eines Lippenstifts, eines Lippenbalsams, eines Abdeckstifts, einer Grundierung, eines Rouges oder eines Lidschattens aufweist.

12. Kosmetisches Verfahren für Haut- oder Lippenpflege oder -Makeup, welches das Auftragen einer Zusammensetzung nach einem der Ansprüche 1 bis 11 umfasst.

13. Verfahren zur Herstellung einer Zusammensetzung nach einem der Ansprüche 1 bis 11, **dadurch gekennzeichnet, dass** es mindestens die folgenden Schritte umfasst:
- Mischen und Erwärmen des Wassers und des Tensids, um eine wässrige Phase zu erhalten,
- Schmelzen der Fettphase, die das Amid und das Alkylvinyl-(Co)-Polymer enthält,
- Einführen der wässrigen Phase in die geschmolzene Fettphase, um ein Fluidgemisch zu erhalten, und
- Abkühlenlassen des Fluidgemischs bis auf Umgebungstemperatur, um eine Zusammensetzung in fester oder cremiger Form zu erhalten.

## Claims

1. A cosmetic skin or lip care or makeup composition in the form of a water-in-oil emulsion comprising a fatty phase, a surfactant, and water, **characterized in that** said fatty phase comprises at least one amide of glutamic acid, said amide comprising at least one alkoyl group having 6 to 14 carbon atoms, and comprises at least one alkyl vinyl (co)polymer comprising at least one alkyl substituent comprising 10 to 30 carbon atoms, the fatty phase comprising at least one first, hydrocarbon nonvolatile oil and at least one second nonvolatile oil comprising at least one ester function and/or at least one alcohol function and at least one hydrocarbon chain comprising 10 to 22 carbon atoms.

2. The composition as claimed in claim 1, **characterized in that** the amide of glutamic acid is selected from dibutyl lauroyl glutamide and dibutyl ethylhexanoyl glutamide.

3. The composition as claimed in claim 1 or 2, **characterized in that** the amide or mixture of amides represents between 0.1% and 15.0%, preferably between 0.5% and 8%, and more preferably between 0.8% and 5% by mass of the mass of the composition.

4. The composition as claimed in one of the preceding claims, **characterized in that** the alkyl vinyl (co)polymer is obtained by polymerization of a monomer comprising at least one alkyl group having 10 to 30 carbon atoms, said monomer being selected from an alkyl(meth)acrylic acid or esters thereof; an alkyl (meth)acrylate; and an alkyl-pyrrolidone.

5. The composition as claimed in one of the preceding claims, **characterized in that** the INCI name of the alkyl vinyl (co)polymer is selected from ACRYLATES/DIMETHICONE METHACRYLATE COPOLYMER, ACRYLATES/BEHENYL ACRYLATE/DIMETHICONE METHACRYLATE COPOLYMER, ACRYLATES/STEARYL ACRYLATE/DIMETHICONE METHACRYLATE COPOLYMER, C10-C30 ALKYL ACRYLATE, VP/EICOSENE COPOLYMER, VP/HEXADECENE COPOLYMER ET TRIACONTANYL PVP.

6. The composition as claimed in one of the preceding claims, **characterized in that** the alkyl vinyl (co)polymer represents between 1% and 30% by mass of the mass of the composition.

7. The composition as claimed in one of claims 1 to 6, **characterized in that** the ratio by mass between the first nonvolatile oil and the second nonvolatile oil is between 1/3 and 3/1.

8. The composition as claimed in one of claims 1 to 7, **characterized in that** it comprises from 15 to 20% by mass of the first nonvolatile oil, from 8 to 15% by mass of the second nonvolatile oil, and from 8 to 12% by mass of a volatile oil.

9. The composition as claimed in one of claims 1 to 8, **characterized in that** the water represents from 10% to 35% by mass of the mass of the composition.

10. The composition as claimed in one of claims 1 to 9, **characterized in that** it comprises an amount of wax(es) of less than 5% by mass.

11. The composition as claimed in one of claims 1 to 10, **characterized in that** it takes the form of a stick such as a lipstick, a lip balm, a corrective stick, a foundation, a blusher or an eyeshadow.

12. A cosmetic method for skin or lip care or makeup, which comprises applying a composition as claimed in one of claims 1 to 11.

13. A method for manufacturing a composition as claimed in one of claims 1 to 11, **characterized in that** it comprises at least the following steps:
- the water and the surfactant are mixed and heated to give an aqueous phase,
- the fatty phase comprising the amide and the alkyl vinyl (co)polymer is melted,
- the aqueous phase is introduced into the melted fatty phase, to give a fluid mixture, and
- the fluid mixture is left to cool to ambient temperature, to give a composition in solid or creamy form.
